# EUROPEAN PATENT APPLICATION

(11) **EP 1 400 174 A1**
(43) Date of publication of application: **24.03.2004**
(21) Application number: 01271881.3
(22) Date of filing: 25.12.2001
(51) Int. Cl.: A23C 3/00, A23C 9/14, A61K 39/00, A61P 31/00, A23K 1/16

(54) **METHOD OF STERILIZING ANTIBODY-CONTAINING MILK AND PRODUCTS CONTAINING STERILIZED ANTIBODY-CONTAINING MILK**

(30) Priority: 26.12.2000 JP 2000394271
(71) Applicant: OGAWA & CO., LTD., Chuo-ku, Tokyo 103-0023 (JP); MORINAGA & CO. LTD., Tokyo 108-0014 (JP)
(72) Inventor: MIHARA, Satoru, Arakawa-ku, Tokyo 116-0013 (JP); SHODA, Masaki, Matsudo-shi, Chiba 270-2203 (JP); HASHIZUME, Shuichi, Yokohama-shi, Kanagawa 236-0005 (JP); KAMEI, Masanori, Yokohama-shi, Kanagawa 230-0077 (JP); SUZUKI, Tatsuo, Bunkyo-ku, Tokyo 112-0001 (JP)
(74) Representative: Bjerre, Nils B.J.
(86) International application number: PCT/JP2001/011376
(87) International publication number: WO 2002/051254

(57) **Abstract**

The invention provides a method for removing bacteria from antibody-containing milk that has been expressed from a bovid animal'producing antibodies by sensitization with one or more antigens selected from among pathogens and their metabolic products, characterized in that defatting treatment is followed by filtration using amicrofilter comprising a support and a filter membrane formed from ceramic fine particles, having filtration pores and a thickness between 40 to 150 µm.

The invention also provides a filter sterilization method which maintains the antibody activity of antibody-containing milk while efficiently removing bacteria fromthemilktoanearlyasepticlevel, as well as immunoactivating agents, foods, beverages and the like that comprise antibody-containing milk wherefrom bacteria is removed by the method as an effective ingredient thereof.

## Description

### Technical Field

The present invention relates to a method for removing bacteria from antibody-containing milk by filtration (i.e. sterile-filtration or filter-sterilization), and to immunoactivatingagents, as well as foods, beverages, nutritional supplements, fodders, feeds, drugs, quasi-drugs and cosmetics that comprise antibody-containing milk wherefrom bacteria is removed by the method.

### Background Art

Antibody-containing milk (also known as "immune milk" or "immunized milk") is produced from milk of cows immunized with vaccines prepared from any of 26 different types of bacteria, as disclosed, for example, in Japanese Unexamined Patent Publication SHO No. 54-113425 relating to suppression of combined gastrointestinal bacterial infection, and Japanese Unexamined Patent Publication SHO No. 57-188523 relating to anti-inflammatory agents, and it is used widely as a natural food not unlike human mother's milk.

Because such antibody-containing milk contains in highly active form antibodies that neutralize numerous human-infecting bacteria, as well as other functional components such as anti-inflammatory factors, it is well-known to be effective for rheumatism, hypertension, hypercholesteremia, allergies, constipation and the like and to bolster immune strength in the elderly and cancer patients.

The possibility of preventing rotavirus infection by oral ingestion of antibody-containing milk has also been investigated. Oral administration of anti-rotavirus antibodies has an effect of preventing the virus infection, and a certain degree of curative effect may be expected if the antibodies have a high titer and neutralizing activity (T. Ebina et al., Med. Microbiol. Immunol., 174, 177(1985), H. Hilpert et al., J. Infect. Dis., 156, 158(1987)).

One of the present inventors (Suzuki et al.) has recently suggested the possibility that the protective function of the intestinal mucosa may be increased by passive immunity conferred through antibody-containing milk obtained by immunization with antigens from 6 different enteroviruses (CVA9, CVA16, CVB3, CVB5, E11 and E18), and especially CVB3, which is well known to produce myocarditis and atrial myocarditis in mice (Suzuki et al., The Journal of the Japanese Association for Infectious Diseases, Vol.73, No.2, pp.122-129(1998)).

As mentioned above, antibody-containing milk promises to be highly useful for prevention or treatment of a variety of diseases. However, since expressed antibody-containing milk contains large amounts of sundry bacteria, it cannot be consumed directly or added to food products and must be passed through the same heat sterilization or sterile-filtration required for general milk production.

Heat sterilization has also been applied as for general milk production. However, when the disappearance of antibody activity in heat-sterilized milk is examined, it has been found that antibody activity falls to about 60-80% with low-temperature sterilization (holding pasteurization) at 63°C for 30 minutes, while antibody activity completely disappears with Ultra-High Temperature (UHT) heating at 130°C for 2 seconds or longer (the sterilization method used for ordinary marketed milk). Consequently, heat sterilization of antibody-containing milk must be carried out by low-temperature-sterilization for food or beverage purposes (Japanese Unexamined Patent Publication HEI No. 4-66050; Ota M., Kagaku to Seibutsu, 37 (2), 107-112 (1999)).

Yet, because low-temperature-sterilization cannot always achieve adequate removal or elimination of bacteria, and proliferation of residual bacteria is therefore a risk, the preservation quality has been a difficult issue to resolve, while public health problems such as Johne's disease have also arisen.

Sterile-filtration has also been attempted since it does not depend on heating, and it can remove or eliminate bacteria with sizes of 1-5 µm. However, even by using screen filter-type membrane filters with pore sizes of 1 µm and smaller it has not been possible to achieve adequate removal of bacteria, while reducing the filtration pore size even further tends to result in serious practical problems such as clogging or fouling.

It has therefore been desirable to develop new sterile-filtration methods which have excellent permeability to antibodies while also achieving thorough elimination and avoiding filter clogging.

### Disclosure of the Invention

It is an object of the present invention to provide a filter-sterilization method which maintains the antibody activity of antibody-containing milk while efficiently removing bacteria in the milk to a nearly aseptic level.

It is another object of the invention to provide immunoactivatingagents, as well as foods, beverages, nutritional supplements, fodders, feeds, drugs, quasi-drugs and cosmetics that comprise antibody-containing milk wherefrom bacteria has been removed by filtration but retains the antibody activity.

As a result of ardently examining pore sizes and also filter layer structures of filters used for removal of bacteria, the present inventors have completed this invention upon finding that antibody-containing milk which is aseptic or nearly aseptic, on a level comparable to heat sterilization, can be obtained by filter-sterilization using a specific microfilter.

In other words, the present invention relates to a method for removing bacteria from antibody-containing milk, which is a method for sterile-filtration treatment of antibody-containing milk that has been expressed from bovid animals producing antibodies by sensitization with one or more antigens selected from among pathogens and their metabolic products, the method being characterized in that defatting treatment is followed by filtration using a microfilter comprising a support and a filter membrane formed from ceramic fine particles, having filtration pores and a thickness between 40 to 150 µm.

The invention further relates to the aforementioned filtration method characterized in that the mean particle size of the ceramic fine particles is between 0.2 to 2.0 µm, the mean pore size of the filtration pores is between 0.4 to 2.0 µm, and the microfilter is a crossflow system.

The invention still further relates to the aforementioned filtration method wherein the microfilter is Sterilox MF (trademark of Societe des Ceramiques Techniques, France).

The invention still further relates to the aforementioned filtration method wherein the pathogen is a bacterium or virus which causes a gastrointestinal or respiratory disease.

The invention still further relates to an immunoactivating agent comprising antibody-containing milk wherefrom bacteria is removed by the aforementioned filtration method as an effective ingredient thereof.

The invention still further relates to foods, beverages, nutritional supplements, fodders, feeds, drugs, quasi-drugs and cosmetics comprising antibody-containing milk wherefrom bacteria is removed by the aforementioned filtration method as an effective ingredient thereof.

The invention will now be explained in greater detail.

### (1) Antibody-containing milk

This is milk that has been expressed (by hand or pump) from a female bovid animal which has received booster doses using as the antigen a pathogen such as a bacterium, virus, fungus, rickettsia or the like (particularly a bacterium or virus which infects the gastrointestinal tract or respiratory system), or a metabolic product thereof.

Preferred as antibody-containing milk is milk expressed from a female bovid animal which has been sensitized by administration of a vaccine prior to administration of a sufficient dose of the sensitized bacterium, virus or metabolic product, as the antigen.

According to the invention, the antigen used to sensitize the female bovid may be the pathogen itself, such as the bacterium or virus which causes infection, or a vaccine containing one or more of its metabolic products, in a harmless (non-active) form.

As bacteria to be used as antigens there maybe mentioned Staphylococcus aureus, Staphylococcus epidermidis, Streptococcus pyogenes, Aerobacter aerogenes, Escherichia coli, Salmonella enteritidis, Pseudomonas aeruginosa, Klebsiella pneumoniae, Salmonella typhimurium, Streptococcus viridans, Proteus vulgaris, Shigella dysenteriae, Streptococcus Group B, Diplococcus pneumoniae, Streptococcus mutans, Corynebacterium Acne, and the like.

As viruses there may be mentioned enterovirus, rotavirus, foot and mouth virus, influenza virus and the like, but the invention is not limited to these or to any other viruses or types.

As female bovid animals to be immunized with antigen-prepared multivalent vaccines containing any of the a forementioned bacterial and viral antigens there may be mentioned cows, goats, sheep and the like, with no particular restrictions.

The amount of bacterial antigen to be administered as antigen will normally be in a number of 1 x 10⁶ to 1 x 10²⁰, and preferably 10⁸ to 10¹⁰.

The amount of a virus used for immunization may be in the range of 0.001-100 mg/inoculation, and preferably 0.1-10 mg/inoculation.

The immunization according to the invention may be conducted according to an ordinary procedure. The immunization will normally be carried out by injecting a prescribed amount of antigen into a suitable part of the bovid animal at a prescribed interval. The immunization is carried out at a prescribed immunization interval, with blood sampling and measurement of the antibody titer, and it is complete when the desired antibody titer has been reached. The antigen must be repeatedly administered with booster shots in order to increase the antibody titer; the immunization interval is the interval at which the antigen is administered for booster shots.

The milk expressed from the antibody-producing bovid animal (antibody-containingmilk) is, if desired, detoxified for removal of bacterial toxins that exhibit toxic activity in humans.

Bacterial toxins, especially pyrogenic toxins, comprise gram-negative bacteria-derived endotoxins which are typically lipopolysaccharides, and gram-positive bacteria-derived exotoxins, which are typically superantigens.

Such bacterial toxins are causes of food poisoning and fever, and in severe cases canprovokeshockordeathofthepatient.

Endotoxins can be removed to some degree by filtration with a reverse osmotic membrane or ultrafiltration membrane, but because separation of endotoxins and antibodies is difficult, membrane filtration methods are generally not applicable for antibody-containing milk.

On the other hand, methods of removing endotoxins by adsorption have been conventionally known which employ for adsorption of the toxins such materials as beads obtained by reacting hexamethylene diisocyanate with fiber-crosslinked agarose beads immobilizing antibiotics such as Polymyxin B (Japanese Unexamined Patent Publication HEI No. 4-114661, "Toraymyxin" by Toray), materials immobilizing histidine or its derivatives (for example, amino acids, iminodiacetic acid or nitrogen-containing heterocyclic compounds (Japanese Unexamined Patent Publication SHO No. 54-183172 and others)), and materials composed of hyaluronic acid and anionic resins (Japanese Unexamined Patent Publication SHO No. 54-67024), and they may be used without anyparticular restrictions so long as thematerial used adsorbs the gram-negative bacteria-derived endotoxin.

Known gram-positive bacteria-derived exotoxins include exotoxins derived from Staphylococcus aureus (enterotoxins A, B and C, toxic shock syndrome toxin-1). Most are characterized by being secreted proteins. As structures for adsorbing such exotoxins there may be used any conventionally known compounds, for example, urea bond- or thiourea bond-containing compounds (Japanese Unexamined Patent Publication HEI No. 10-85330, Japanese Unexamined Patent Publication HEI No. 10-147533, Japanese Unexamined Patent Publication HEI No. 10-225515), without any particular restrictions so long as they adsorb gram-positive bacteria-derived endotoxins.

### (2) Defatting treatment

The fat globule diameter distribution in the pumped antibody-containing milk will normally be from 0.1-22 µm, with an average of 3 µm. The sizes of the various bacteria cells to be eliminated are usually 1-5 µm, as mentioned above, and this is comparable to the sizes of fat globules. Since it is therefore difficult to separate them based on size using a filtration membrane, defatting treatment is necessary in a prior step of precision filtration with a microfilter, whereby a centrifuge or the like is used for separation of the fat globules from the milk antibodies and casein protein (0.04-0.3 µm) based on differences in the specific gravities.

### (3) Filter-sterilization treatment

Skim milk with a milk fat content of 0.1% or lower obtained by defatting treatment is subjected to filter-sterilization using a specific microfilter as described below, to produce filter sterilized antibody-containing milk.

The microfilter is composed entirely of a ceramic of aluminum oxide or the like, including the support supporting the filter membrane. The filter section is a filter membrane formed from aggregated ceramic fine particles, having filtration pores and a thickness between 40 to 150 µm, or preferably between 60 to 100 µm. A filter membrane of such a thickness canbe fabricated, for example, by laminating two, three or more thin filter membranes.

The microfilter is preferably a crossflow system, wherein the direction of supply liquid (concentrated liquid) flow and the direction of permeating liquid flow are orthogonal.

The mean particle size of the ceramic fine particles of the microfilter is preferably between 0.2 to 2.0 µm and especially between 0.4 to 1.8 µm, and the mean pore size of the filter is between 0.4 to 2.0 µm, and especially between 0.8 to 1.6 µm, in order to both achieve improved filter sterilization efficiency and prevent clogging.

Sterilox MF™ manufactured by Societe des Ceramiques Techniques (SCT), France may be purchased and used as a microfilter satisfying these conditions.

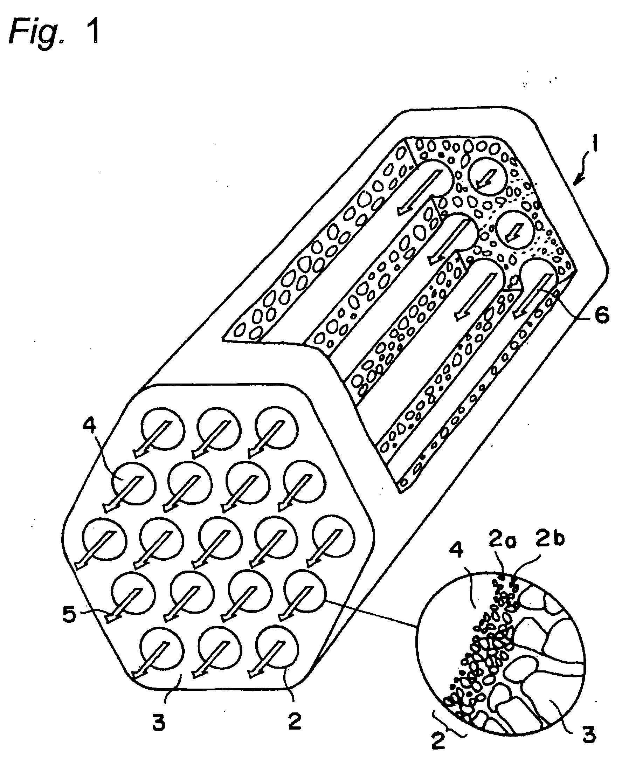

The structure of a preferred microfilter for the method of the invention is shown in Fig. 1.

Fig. 1 is a partially sectional perspective view of amicrofilter, also showing the internal structure, together with amagnified section of the cross-sectional structure of the filter membrane. The microfilter (1) comprises a plurality of channels (4) for a supply liquid to flow in the direction of the arrows, where the interface surrounding each of the channels (4) comprises a filter membrane layer (2) formedbylaminatinga filtermembrane second layer (2a) and a filter membrane first layer (2b) composed of minute ceramic fine particles and having filtration pores, and a coarse ceramic support (3) which supports it. The antibodies and casein protein in antibody-containing milk (6) flowing into the channels (4) pass through the filter membrane (2) , while the concentrate (5) containing a large abundance of various sundry bacteria is discharged to separate those bacteria. The concentrate may also be recirculated for a second or more times filtration.

The sizes of common bacteria are generally 1-5 µm, and since the vacant space or gaps in the filter membrane are not consistent and the membrane has a thickness of 40-150 µm, even bacteria which are smaller than the filtration pores cannot pass through the filter and are thus separated from the antibody-containing milk.

Also, by using a crossflow type filter wherein the milk is caused to flow at high speed parallel to the surface of the filter membrane so that the permeating liquid passing through the membrane and the concentrate that does not pass through are separated, it is possible to avoid clogging of the microfilter by bacteria and thus achieve high filter-sterilization efficiency and prolonged stable filter- sterilization.

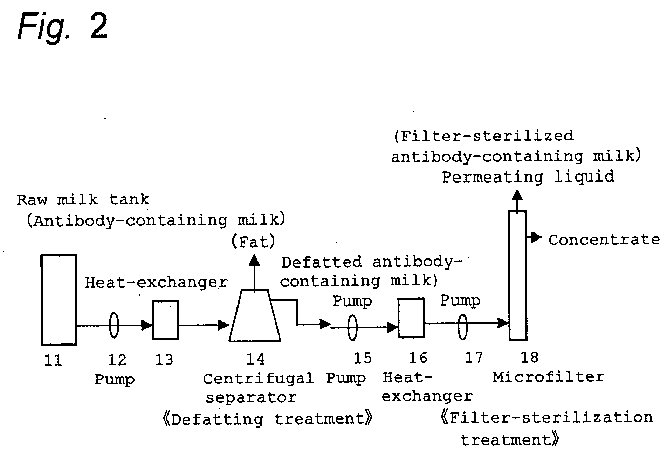

Fig. 2 is a process diagram for an example of filter-sterilization treatment. Antibody-containing milk is supplied from a milk tank (11) by a pump (12) and heated to a suitable separation temperature at a heat-exchanger (13), and it is then fed to a centrifugal separator (14) to separate the fat and the skim milk. The skim milk is then heated to a suitable separation temperature at a heat exchanger (16) by the action of a pump (15) , and then fed to a microfilter (18) through a pump (17). The permeating liquid passing through the filter membrane is the bacteria-removed milk. The concentrate that does not pass through the membrane is bacteria-abundant milk.

In the case of a crossflow filtration system, the force of the milk permeating the filter membrane depends on the difference between the pressure of the permeating liquid side and the pressure of the concentrated liquid side (trans-membrane pressure), and this creates an overall pressure loss for the membrane. Experience has shown that operation with a smaller pressure difference gives superior performance, such as delayed clogging of the membrane. Consequently, in order to achieve operation under ideal conditions for the membrane as a whole when using a crossflow type microfilter, it is preferred to operate the filtration apparatus with "uniform trans-membrane pressure" (UTP), by filling the channels on the permeating liquid side, which are normally hollow, with small plastic spheres, and by adjusting the pressure on the permeating liquid side with a pump to produce high-speed circulation in aparallel flow on the outside of the filtration membrane as on the concentrated liquid side so that the pressure difference between the pressure on the concentrated liquid side and the pressure on the permeating liquid side is uniform throughout the entirety of the channels of the microfilter.

Furthermore, since most substances such as microparticles and microbes have unique ζ (zeta) potentials when immersed in water, the microfilter used may be a filter with a ζ potential to also obtain an electrical adsorption effect.

The filtering step with a microfilter may be an appropriate method commonly carried out for microfiltering treatment, such as a method of filtering the antibody-containing milk with the microfilter once, a method of refiltering the microfilter permeating liquid with the microfilter either once or several times, or a method of returning the concentrate to the raw material side for recirculation to the microfilter.

When it is desired to maintain a high filter-sterilization rate, the target may be achieved by repeated filtration.

The temperature of the milk during the filter-sterilization treatment is preferably 40-55°C, and especially 45-50°C. Heating the milk facilitates deformation of the fat globules to ease their permeation. If the heating temperature is below 40°C the effect maybe insufficient, while if it exceeds 55°C the components may undergo alteration, and therefore heating for microfiltering treatment is preferably followed by cooling of the permeating liquid as quickly as possible.

The filter-sterilized antibody-containing milk may be stored for long periods, such as 1 year or longer, in the form of a powder while maintaining a high antibody titer.

The method of powder preparation is preferably freeze-drying (FD) without heat treatment to avoid lowering the antibody titer, or reduced pressure spray drying at 50 °C or below.

The liquid or powdered filter sterilized antibody-containing milk obtained in this manner may be mixed with or added to various types of raw materials as appropriate to produce immunoactivating agents, as well as antibody-containing foods, beverages, nutritional supplements, fodders, feeds, drugs, quasi-drugs and cosmetics.

### (4) Immunoactivating agent

An immunoactivating agent according to the invention may be used for prevention, treatment or remission of infectious diseases. The dosage form of the immunoactivating agent may be any dosage form which is publicly known in the field of medicine. For example, it may be in the form of a liquid formulation, or included in tablets, capsules (preferably enteric coated), powder, a sol, a gel, granules or liposomes.

For preparation of a formulation, any of various excipients or additives publicly known to those skilled in the art maybe used in pharmaceutically acceptable amounts. The route of administration may be, for example, oral, intravenous, subcutaneous, intramuscular, intraabdominal, intranasal, intrapharyngeal, etc. depending on the dosage form and manner of preparation.

For patients with conditions not suitable for oral ingestion, the agent may be used as a nutritional supplement by per anum administration or oral tube, or directly into the bowel.

### (5) Utilization in foods, etc.

Antibody-containing milk from which bacteria has been removed in the manner described above may be added to foods and the like to increase their health value.

The foods may be in the form of solids, liquids, sols, gels, powders, granules or the like, andmaybeproducedbymethods which are publicly known in the relevant technical fields. These include, for example, cakes, candies, Japanese or Chinese confectioneries, frozen desserts, yogurts, fats and oils, dairy creams, pastes, livestock (or fish) products, fishpasteproducts, delicacies, canned goods, boiled fish or vegetables, processed meats, noodles, flavorings and the like.

Beverages to which the milk may be added include milk and fruit drinks, as well as dairy beverages such as liquid yogurt, lacticacidbacteriabeverages (fordirectconsumption) andacidic milk beverages (for dilution) , rice-malt beverages such as sweet drink made from fermented rice, bean-based beverages such as soy milk, coffee and cocoa, leaf-based beverages such as unfermented tea (green tea), semi-fermented tea (ulong tea) and fermented tea (black tea), and carbonated beverages.

Drugs may be used in any desired form which is publicly known in the relevant technical fields. These include, for example, liquid formulations, or forms which are included in tablets, capsules (preferably enteric coated), powders, sols, gels, granules or liposomes.

For preparation of a formulation, any of various excipients or additives publicly known to those skilled in the art may be used in pharmaceutically acceptable amounts. The route of administration may be, for example, oral, intravenous, subcutaneous, intramuscular, intraabdominal, intranasal, intrapharyngeal, etc. depending on the dosage form and manner of formulation.

For patients with conditions not suitable for oral ingestion, the agent may be used as a nutritional supplement by per anum administration or oral tube, or directly into the bowel.

Quasi-drugs are defined according to the Pharmaceutical Affairs Law of Japan, Article 2 and include breath fresheners, anti-hircismus agents, dusting powders, hair growth promoters (hair growth stimulants), hair removers, hair dyeing agents, bath agents, medicinal cosmetics, medicinal dental pastes and the like.

Antibodies against cavity-causing bacteria, for example, may be used in oral compositions such as toothpastes, mouthwashes or chewing gum.

They may also be mixed with feeds, fodders, drugs and the like as highly active immunoactivating agents for breeding animals such as livestock, poultry and fish.

Anti-keratin antibodies (H. Uchiwa: J. Soc. Cosmet. Chem., 48, 209(1997)) or antibodies against acne-causingbacteria may also be added to cosmetics and pharmaceutical products.

Aromatics according to the invention include aromatics for foods, nutritional supplements, fodders, feeds, drugs, quasi-drugs and cosmetics. Such aromatics may be used in the form of essences (water-soluble fragrances), oils (oil-soluble fragrances), aromatic emulsions, powdered aromatics and the like.

Antibody-containing milk obtained by the filter-sterilization method of the invention may be added to a food or beverage in any step up to completion of production of the food or beverage, and the addition may be accomplished by a method appropriately selected among those that are publicly known, such as mixture or dispersion at a low temperature of 50°C or below, for example.

After addition of the antibody-containing milk, it is cooled to below room temperature as quickly as possible.

An immunoactivating agent according to the invention may be used against various types of infection in such forms as foods, nutritional supplements, fodders, feeds, drugs, quasi-drugs and cosmetics. As examples of target infections there may be mentioned respiratory infections, oral infections, gastrointestinal infections, laryngeal infections and the like, as well as any other types of infection.

The filter-sterilized antibody-containing milk (antibodies) may be ingested in an amount of 1-20 g and preferably 5-10 g per kilogram of body weight per day. For example, when ingested as a health food, 45 g thereof is preferably diluted 5-fold with water at 50°C (a 225 g aqueous solution) and consumed in the same manner as ordinary milk twice a day, in the morning and in the evening (450 g milk solution/60 kg body weight = 7 g milk solution/kg body weight).

### Brief Description of the Drawings

Fig. 1 is a partially sectional cross-sectional perspective view of a preferred microfilter for the invention.
Fig. 2 is a process diagram for an example of preferred filter-sterilization treatment of antibody-containing milk according to the invention.

The reference numerals in Fig. 1 and Fig. 2 represent the following.
- 1,18: : Microfilter
- 2: : Filter membrane
- 2a: : Filter membrane second layer
- 2b: : Filter membrane first layer
- 3: : Support
- 4: : Channels
- 5: : Concentrate
- 6: : Antibody-containing milk
- 11: : Raw milk tank
- 12,15,17: : Pumps
- 13,16: : Heat-exchanger
- 14: : Centrifugal separator

### Best Mode for Carrying Out the Invention

The present invention will now be explained in greater detail through examples and comparative examples, with the understanding that these examples are not intended in any sense to be limitative on the invention.

### Example 1: Antibacterial antibody-containing milk

### (1) Preparation of multivalent antigen

The bacteria were cultured (37°C, 48 hours) and then the medium was heated at 60°C for 2 hours for sterilization. The sterile medium was rinsed with distilled water and the cells were collected by centrifugal separation. The sterilized bacterial cells were mixed to prepare a multivalent antigen.

**Table 1**

| Bacterial antigens | |
|---|---|
| Bacteria | American Type Culture Collection No |
| *Staphylococcus aureus* | 11631 |
| *Staphylococcus epidermidis* | 155 |
| *Streptococcus pyogenes A1* | 8671 |
| *Streptococcus pyogenes A3* | 10389 |
| *Streptococcus pyogenes A5* | 12347 |
| *Streptococcus pyogenes A8* | 12349 |
| *Streptococcus pyogenes A12* | 11434 |
| *Streptococcus pyogenes A14* | 12972 |
| *Streptococcus pyogenes A18* | 12357 |
| *Streptococcus pyogenes A22* | 10403 |
| *Aerobacter aerogenes* | 884 |
| *Escherichia coli* | 26 |
| *Salmonella enteritidis* | 13076 |
| *Pseudomonas aeruginosa* | 7700 |
| *Klebsiella pneumoniae* | 9590 |
| *Salmonella typhimurium* | 13311 |
| *Haemophilus influenzae* | 9333 |
| *Streptococcus viridans* | 6249 |
| *Proteus vulgaris* | 13315 |
| *Bacillus dysenteriae* | 11835 |
| *Streptococcus B* | |
| *Pneumococcus* | |
| *Streptococcus mutans* | |
| *Corynebacterium acne 1* | |
| *Corynebacterium acne 2* | |

### (2) Production of antibacterial antibody-containing milk

The polyvalent antigen prepared in (1) above (5 ml bacteria, 20 x 10⁸ cells/ml) were administered to each of healthy cows by the procedure described below, and milk was collected from the immunized cows from the 5th week after initial administration of the antigen.

### [Antigen administration procedure]

i) Administration method: Intramuscular injection
ii) Administration interval: Once per week from the 1st to 4th weeks after initial administration, followed by twice per month from the 5th week onward.

### (3) Filter-sterilization of antibacterial antibody-containing milk

The following explanation is based on Fig. 2.

On the same day that the antibacterial antibody-containing milk in (2) above was obtained, 500 liters thereof shipped to the dairy factory was supplied from a tank (11) to a heat-exchanger (13) with a pump (12) and heated at 48°C.

A centrifugal separator (14) (Model 29AE by Tetra Pak) was then used for defatting treatment at 48°C with a centrifugal force of 7400 g (Max: 7440 G) to remove the fat portion.

Next, 20 liters of the defatted antibody-containing milk was supplied to a heat-exchanger (16) with a pump (15) and heated at 48°C, after which a pump (17) was used to feed it into a microfilter mounted on a filtration apparatus ("Tetra Alcross^{R} M" Precision Filtration System, by Tetra Pak).

The microfilter was a "Sterilox MF" ceramic filter manufactured by Societe des Ceramiques Techniques, France, having the following properties.

| Sterilox MF properties | |
|---|---|
| System | Crossflow system |
| Material | Aluminum oxide (support and filtration membrane) |
| Membrane area | 0.2 m² |
| Channel length | 85 cm |
| Filtration membrane | Double layer structure filter with 70 µm thickness and comprising aluminum oxide fine particles (mean particle size: 1.5 µm) |
| Membrane average pore size | 1.4 µm |

The filtration was accomplished by moving the antibody-containing milk at high speed along the filter surface at a treatment temperature of 50°C and a filter permeating flow rate of 410 liter/h·m².

The apparatus was operated for approximately 13 minutes, and 2 liters of bacteria-abundant concentrate was continuously removed to obtain 18 liters of permeating liquid (filter-sterilized antibacterial antibody-containing milk).

### Example 2: Antiviral antibody-containing milk

### (1) Preparation of antigen used for antiviral antibody-containing milk

The antigen was prepared in the same manner as described by Suzuki et al. in "The Journal of the Japanese Association for Infectious Diseases" (Vol.73, No.2, pp.122-129(1998)). Specifically, Coxsackie virus A9 (CVA9) (Bozek strain) was used to infect LLC-MK2 cells, which were then cultured for 2 days using 2% fetal bovine serum (FBS) -containing minimum essential medium (MEM, product of GIBCO BRL).

After adding 30% polyethylene glycol and 0.4% NaCl to the culture supernatant obtained by centrifugation at 10,000 g for 30 minutes, the mixture was stationed at 4°C for 16 hours.

After further centrifugationat 10, 000 g for 30minutes, the precipitate was resuspended in an STE solution [0.15 M NaCl, 0.15 M Tris-HCl, 0.15 M EDTA-2Na (pH 7.4)]. The suspension was then superposed onto a 30%/60% (W/V) sucrose non-continuous gradient and subjected to centrifugation at 100,000 g, 4°C for 2.5 hours.

The band at the 60% sucrose interface was collected and inactivated with β-propiolactone (Wako Pure Chemical Industries Co., Ltd.), as the viral antigen for antibody-containing milk.

### (2) Production of antiviral antibody-containing milk

Female cows were each intradermally inoculated with 10 ml of a combination of the viral immunogen (0.5 mg/ml protein) with Freund's complete adjuvant (Sanko Junyaku Co., Ltd.).

The immunizationwas carriedout at two-week intervals, and the cows were impregnated after confirming serum antibody level increase by ELISA.

After birth, milkwas collected from the immunized cows to obtain antiviral antibody-containing milk with a fat content of 3.8%.

### (3) Filter sterilization of antiviral antibody-containing milk

Twenty liters of the antiviral antibody-containing milk obtained in (2) above was subjected to filter-sterilization treatment in the same manner as Example 1, and 18 liters of permeating liquid (antiviral filter-sterilized antibody-containing milk) was obtained.

### Comparative Example 1: Heat sterilization of antibacterial antibody-containing milk

The antibacterial antibody-containing milk obtained in Example 1, prior to filter-sterilization, was subjected to different heat sterilization procedures by low-temperature sterilization (63°C, 30 minutes), high-temperature, short-time (HTST) sterilization (77°C, 15 seconds) and ultrahigh-temperature (UHT) heat sterilization (150°C, 1 second).

### [Residual bacteria test]

Residual bacteria-measuring samples of the antibacterial antibody-containing milk obtained in Example 1 were taken from the septum on the microfilter-permeating liquid side and the concentrate side in a sealed system using a syringe (3 times in total, 3 samples), and the samples were subjected to a residual bacteria test under the following culturing conditions [1] to [3].

The heat sterilized antibody-containing milk obtained in Comparative Example 1 and completely unsterilized antibody-containing milk were also subjected to the same test.

Bacterial growth was judged to be positive if turbidity was observed in clinical thioglycolate (TGC) medium after 2 weeks from the start of culturing. Bacterial growth was also judged to be positive if colony growth was observed on heart infusion (HI) agar medium. The results are shown in Table 2.

### [Culturing conditions]

### [1] 100 ml glass vial culturing test ("100 ml TGC" in Table 2)

There were prepared two 150 ml glass vials containing 100 ml of clinical thioglycolate (TGC) medium and 10 ml of the specimen. Culturing was carried out for 2 weeks at a temperature of 30°C for one vial and 37°C for the other vial, and the presence of turbidity in the liquid medium was observed.

### [2] 10 ml test tube culturing test ("10 ml TGC" in Table 2)

There were prepared two 30 ml test tubes containing 10ml of clinical thioglycolate (TGC) medium and 1ml of the specimen. Culturing was carried out for 2 weeks at a temperature of 30°C for one test tube and 37 °C for the other test tube, and the presence of turbidity in the liquid medium was observed.

### [3] Heart infusion agar medium culturing test ("HI agar medium" in Table 2)

After dropwise addition of 0.1 ml of the specimen to heart infusion (HI) agarmedium, it was cultured at 37°C and growth of the colonies was observed daily.

### [Immunoglobulin (IgG) residue test]

Table 2 shows the IgG residue rates for the filter sterilized antibody-containing milk of Example 1, the heat-sterilized antibody-containing milk of Comparative Example 1 and completely unsterili zed antibody-containing milk. The IgG contents were assayed by the immunodiffusion method.

**Table 2**

| Residual bacteria and IgG residue rates | | | | | | |
|---|---|---|---|---|---|---|
| Specimen No. | Culturing at 37°C | | Culturing at 30°C | | Culturing at 37°C | IgG residue rate (%) |
| | 100 ml TGC | 10 ml TGC | 100 ml TGC | 10 ml TGC | HI Agar medium | |
| Filter sterilization (Example 1) | | | | | | |
| 1. Permeating liquid [1] | - | - | - | - | - | 100 |
| 2. Permeating liquid [2] | - | - | - | - | - | 100 |
| 3. Permeating liquid [3] | - | - | - | - | - | 100 |

| Heat sterilization (Comp. Ex. 1) | | | | | | |
|---|---|---|---|---|---|---|
| 4. Low-temperature sterilization (63°C, 30 min) | + | + | + | + | + | 75 |
| 5. HTST sterilization (75°C, 15 sec) | + | + | + | + | + | 75 |
| 6. UHT sterilization (150°C, 1 sec) | - | - | - | - | - | 0 |
| 7. Non-sterilized antibody-containing milk | + | + | + | + | + | 100 |
| (Note) +: bacteria-positive, -: bacteria-negative | | | | | | |

As shown in Table 2, the sterile-filtered antibody-containing milk samples filtered with "Sterilox MF™" in Example 1 (permeating liquids [1], [2] and [3]) were nearly aseptic, and the IgG residue rate was 100%.

Bacterial growth was observed in the unsterilized antibody-containing milk (specimen No.7), and in the low-temperature heat-sterilized (63°C, 30 minutes; specimen No.4) and HTST sterilized (77°C, 15 seconds; specimen No.5) antibody-containing milk.

However, while no bacterial growth was observed in the UHT sterilized (150°C, 1 second; specimen No.6) antibody-containing milk, IgG was also absent. These results indicated that it is difficult to produce aseptic or nearly aseptic "antigen-containing milk with no heat deterioration of IgG" by heat sterilization.

Production of "aseptic or nearly aseptic antigen-containing milk with no heat deterioration of IgG" was only achieved when the defatted antibody-containing milk was subjected to filter-sterilization using "Sterilox MF™".

### Comparative Example 2

The microfilter was changed from "Sterilox MF™" to "Membralox MF™" (ceramic filter by Societe des Ceramiques Techniques, France), and the defatted antibody-containing milk was filter-sterilized under the same conditions as in Example 1. The "Membralox MF" is a single-layer crossflow type filter with half the filter membrane thickness of "Sterilox MF". The permeating flow rate is 480 liter/h·m² (treatment temperature: 50°C). The bacteria removal test results are shown in Table 3. The removal of bacteria was clearly inadequate.

**Table 3**

| Results of bacterial removal test of antibacterial antibody-containing milk using Membralox MF | | | | | | | | | | | |
|---|---|---|---|---|---|---|---|---|---|---|---|
| No. | Filtered antibody-containing milk | Culturing at 37°C | | | | | Culturing at 30°C | | | | |
| | Test tube No.: | 1 | 2 | 3 | 4 | 5 | 1 | 2 | 3 | 4 | 5 |
| [1] | | + | + | + | + | + | + | + | + | + | + |
| [2] | | + | + | + | + | + | + | + | + | + | + |
| [3] | | + | + | + | + | + | + | + | + | + | + |
| [4] | | + | + | - | + | + | + | + | + | + | + |
| [5] | | + | + | - | - | - | + | + | + | + | + |
| [6] | | + | + | + | + | + | + | + | + | + | + |
| [7] | | - | + | + | + | + | + | + | + | + | + |
| [8] | | + | + | + | + | + | + | + | + | + | + |
| [9] | | - | + | + | + | + | - | + | - | + | - |
| (Note 1) +: Growth positive after 14 days of culturing in thioglycolate medium -: Growth negative after 14 days of culturing in thioglycolate medium (Note 2) Test tube Nos. [1]-[9] are the numbers for the filtered out permeating liquid fractions. The number columns 1-5 represent 5 test tubes of samples of the same fraction number, cultured under different conditions, 37°C and 30°C. | | | | | | | | | | | |

### Comparative Example 3

The microfilter was changed from "Sterilox MF™" to the following cellulose acetate filters, and the defatted antibody-containing milk was filter-sterilized under the same conditions as in Example 1.

Membrane Filter Cellulose Acetate (Toyo Roshi Kaisha, Ltd.)
(Diameter: 90 mm, flat)
(4 Pore sizes: 0.45 µm, 0.8 µm, 1 µm and 5 µm)

The cellulose acetate filters were used alone or in combinations for filtration of the defatted antibody-containing milk.

The results are shown in Table 4. Filtration was successfully achieved using the filters with pore sizes of 0.8 µm and larger, but bacteria removal was not adequate. With the pore size of 0.45 µm, clogging occurred and prevented filtration.

**Table 4**

| Bacteria removal test with cellulose acetate filters | | | |
|---|---|---|---|
| No. | Filtration pore size (µm) and combination | Filtration | Elimination |
| [1] | 5 µm | ○ | × |
| [2] | 5 µm, followed by 1 µm | ○ | × |
| [3] | 5 µm, followed by 1 µm, followed by 0.8 µm | ○ | × |
| [4] | 5 µm, followed by 0.45 µm | × | - |
| [5] | 5 µm, followed by 1 µm, followed by 0.45 µm x | × | - |
| [6] | 5 µm, followed by 1 µm, followed by 0.8 µm, followed by 0.45 µm | × | - |
| [7] | 0 filter paper | × | - |
| (Note) o: Filterable ×: Not filterable , -: Not measured | | | |

### Example 3: Production of filter-sterilized antiviral antibody-containing milk-added liquid yogurt

A milk mixture containing raw milk and powdered skim milk was inoculated with 5% of a starter prepared using *Streptococcus thermophilus*, and cultured at 40°C to a pH of 4.2 to obtain liquid yogurt.

Ninety parts by weight of the liquid yogurt and 10 parts by weight of the filter-sterilized antiviral antibody-containing milk obtained in Example 2 were filled into a sterilized container in a sterile filling room to produce antiviral antibody-containing liquid yogurt.

The neutralizing antibody titer of the antiviral antibody-containing liquid yogurt was assayed by the method described below.

### Comparative Example 4: Production of low-temperature sterilized antiviral antibody-containing milk-added liquid yogurt

Ninety parts by weight of the liquid yogurt obtained in Example 3 and 10 parts by weight of the low-temperature sterilized (63°C, 30minutes)antiviral antibody-containing milk (before filter sterilization) obtained in Example 2 were mixed in a sterile filling room and filled into a sterilized can to produce antiviral antibody-containing liquid yogurt.

The neutralizing antibody titer of the antiviral antibody-containing liquid yogurt was assayed by the method described below.

### Example 4: Production of filter-sterilized antiviral antibody-containing milk-added sports drink

Ninety-eight parts by weight of a heat-sterilized sports drink produced with the components and contents shown in Table 5 by a method publicly known to those skilled in the art and 2 parts by weight of the filter-sterilized antiviral antibody-containing milk obtained in Example 2 were filled into a sterilized can in a sterile filling room to produce a sports drink. The units in Table 5 are all parts by weight.

The neutralizing antibody titer of the antiviral antibody-containing sports drink was assayed by the method described below.

Here, "sports drink" refers to a drink whose primary purpose is to supply water or electrolytes lost from the human body by exercise. The first such drink was developed in 1965 in the U.S. to alleviate dehydration sufferedby football athletes performing rigorous exercise. Sports drinks also contain added sugars (3-6%) as an energy source, as well as inorganic salts, flavorings and the like. For more effective absorption of water into the body, the osmotic pressure of sports drinks is often matched to the osmotic pressure of the body, and they are therefore often referred to as "isotonic beverages".

**Table 5**

| Sports drink | |
|---|---|
| Components | Content (wt%) |
| Orange concentrate | 0.200 |
| Sugar | 1.8 |
| Isomerized glucose | 5.5 |
| Citric acid | 0.14 |
| Salt | 0.08 |
| Sodium citrate | 0.07 |
| Potassium chloride | 0.04 |
| Calcium (I) phosphate | 0.013 |
| Monosodium glutamate | 0.004 |
| Magnesium chloride | 0.003 |
| Ascorbic acid | 0.1 |
| Emulsified aromatic | 0.11 |
| Essence | 0.2 |
| Filter-sterilized antiviral antibody-containing milk (Example 2) | 2.0 |
| Water | remainder |
| Total | 100.0% |

### [Assay of neutralizing antibody titer]

LLC-MK2 cells were dispensed into a microplate and neutralization reaction was conducted upon formation of a monolayer. A two-fold stage dilution of each of the food/beverage samples [1] to [4] listed in Table 6 was prepared, and an equivalent volume of diluted virus in a 100-fold concentration tissue culture infectious dose (100 x TCID₅₀/ml) was added prior to reaction at 37°C. After the reaction, the cells were infected and cultured at 37°C, and the degree of dilution in which cell degeneration was observed was recorded as the neutralizing antibody titer. The neutralizing antibody titers for Coxsackie virus A9 (CVA9) are shown in Table 6.

The filter-sterilized antiviral antibody-containing milk exhibited high resistance of 32, 000 (2¹⁵) or greater against CVA9.

The results indicated that production of aseptic or nearly aseptic antibody-containing milk with a high neutralizing antibody titer can be produced by filter- sterilization, and that the neutralizing antibody titer is not reduced even when the milk is added to foods or beverages.

**Table 6**

| Coxsackie virus A9 (CVA9) neutralizing antibody titers | |
|---|---|
| Food/beverage sample | Coxsackie virus A9 |
| [1] Filter-sterilized antiviral antibody-containing milk (Example 2) | 2¹⁵ |
| [2] Filter-sterilized antiviral antibody-containing milk-added yogurt (Example 3) | 2¹² |
| [3] Filter-sterilized antiviral antibody-containing milk-added sports drink (Example 4) | 29 |
| [4] Low-temperature sterilized antiviral antibody-containing milk-added yogurt (Comparative Example 4) | 2⁸ |

### Industrial Applicability

The present invention is industrially applicable as a result of the following advantageous effects.
(1) According to the filter-sterilization method of the invention, it is possible to totally or almost totally eliminate bacteria from antibody-containing milk pumped from bovid animals while maintaining high antibody activity. The sterilization can also be achieved in a highly efficient manner without clogging of the filter even with high-speed filtration.
(2) Immunoactivating agents comprising antibody-containing milk treated by the filter-sterilization method of the invention have high antibody titers and low bacteria contents, and therefore exhibit excellent effects while being highly safe.
(3) Foods, beverages, nutritional supplements, fodders, feeds, drugs, quasi-drugs and cosmetics comprising antibody-containing milk treated by the filter-sterilization method also have high antibody titers and low bacteria contents, and thus exhibit excellent immunoactivating effects while being highly safe. They may therefore be used for light prevention, curing, treatment or alleviation of infection by various pathogens such as bacteria and viruses etc., as well as their metabolic products, and related pathogenesis.

## Claims

1. A method for removing bacteria from antibody-containing milk that has been expressed from a bovid animal producing antibodies by sensitization with one or more antigens selected from among pathogens and their metabolic products, **characterized in that** defatting treatment is followed by filtration using amicrofilter comprising a support and a filter membrane formed from ceramic fine particles, having filtration pores and a thickness between 40 to 150 µm.

2. The method of claim 1, **characterized in that** the mean particle size of the ceramic fine particles is between 0.2 to 2.0 µm, and the mean pore size of the filtration pores is between 0.4 to 2.0 µm.

3. The method of claim 1, **characterized in that** the microfilter is a crossflow system.

4. The method of claim 1, **characterized in that** the microfilter is Sterilox MF (trademark of Societe des Ceramiques Techniques, France).

5. The method of claim 1, **characterized in that** the pathogen is a bacterium or virus which causes a gastrointestinal or respiratory disease.

6. An immunoactivating agent comprising antibody-containing milk wherefrom bacteria is removed by the method of claim 1 as an effective ingredient thereof.

7. A food, beverage, nutritional supplement, fodder, feed, drug, quasi-drug or cosmetic comprising antibody-containing milk wherefrom bacteria is removed by the method of claim 1 as an effective ingredient thereof.
